(19) Europäisches Patentamt
European Patent Office
Office européen des brevets

(11) **EP 1 349 487 B1**

(12) **EUROPEAN PATENT SPECIFICATION**

(45) Date of publication and mention
of the grant of the patent:
**02.09.2009 Bulletin 2009/36**

(51) Int Cl.:
*A61B 3/113* *(2006.01)*     *A61B 3/14* *(2006.01)*
*G06T 5/50* *(2006.01)*

(21) Application number: **01271173.5**

(22) Date of filing: **20.12.2001**

(86) International application number:
**PCT/SE2001/002839**

(87) International publication number:
**WO 2002/049506 (27.06.2002 Gazette 2002/26)**

(54) **IMAGE CAPTURING DEVICE WITH REFLEX REDUCTION**

BILDAUFNAHMEVORRICHTUNG MIT REFLEXREDUKTION

DISPOSITIF DE CAPTURE D'IMAGES A REDUCTION DE REFLEXIONS

(84) Designated Contracting States:
**AT BE CH CY DE DK ES FI FR GB GR IE IT LI LU
MC NL PT SE TR**

(30) Priority: **21.12.2000 SE 0004741**

(43) Date of publication of application:
**08.10.2003 Bulletin 2003/41**

(73) Proprietor: **Smart Eye AB
411 21 Göteborg (SE)**

(72) Inventor: **RYDBECK, Bo
S-414 67 Göteborg (SE)**

(74) Representative: **Edlund, Fabian et al
Awapatent AB
P.O. Box 11394
404 28 Göteborg (SE)**

(56) References cited:
**WO-A1-00/11935**

• **Y. EBISAWA: 'Improved video-based eye-gaze
detection method' IEEE TRANSACTIONS ON
INSTRUMENTATION AND MEASUREMENT vol.
47, no. 4, August 1998, pages 948 - 955,
XP000866987**
• **M. OHTANI ET AL.: 'Eye-gaze detection based on
the pupil detection technique using two light
sources and the image difference method' 1995
IEEE-EMBC AND CMBEC, THEME 7:
INSTRUMENTATION 1995, pages 1623 - 1624,
XP010214956**
• **J.A. LAHOUD ET AL. 4TH ANNUAL 1994 IEEE
DUAL-USE TECHNOLOGIES AND
APPLICATIONS CONFERENCE 1994, SUNY
INSTITUTE OF TECHNOLOGY AT UTICA/ROME,
NEW YORK, pages 308 - 314, XP002966295**

## Description

Technical field

In its most general form, the present invention relates to an image capturing device with reflex reduction, comprising at least one wave emitter for emitting electromagnetic waves towards an object and at least one image capturing device, directed towards the object.

More specifically, the invention relates to a device for eye detection of a person (for example relative position of eyelids, the position of the eyes, iris and eye corners, the bright eye effect etc), also comprising a computer processor, for processing images from said image capturing device of the face of the person and determining the required information from said images.

Technical background

When capturing images, especially images intended for automatic analysis, several problems related to the light conditions are present, making computer treatment of the images difficult. To begin with, in some cases the light conditions are difficult or transient, resulting in the images of an object constantly changing. For instance, the surrounding light, such as sunlight from a window or light from a computer screen, may vary, resulting in consecutive images with very different appearances. In other cases it might be dark, making it necessary to illuminate the object in order to acquire any picture at all. Further, parts of the object may be hidden behind obstacles which affect the light. For example, shaded glass may effectively hide an object. These and other problems can in some cases lead to difficulties in acquiring high quality images or performing a desired analysis of the images. Under these and other circumstances, it is desirable to improve the information in the images by actively exposing the object to electromagnetic radiation (hereafter referred to as *active* radiation), and then capturing the image with the camera. The active radiation can be of a certain wavelength or in a certain wavelength interval, in which case the camera preferably is arranged to only capture incident waves of the determined wavelength, so that variations in the surrounding light conditions therefore interfere less with the image capturing process. The procedure also has the advantage that the camera can "see through" sunglasses, visors and the like. In practice, electromagnetic waves in the IR-region has shown to be quite advantageous, as it is easy to provide and does not disturb the vision of a user.

A problem with this technique occurs, however, when areas of enhanced reflection are present in the object, this often being the case when the object has a varying surface structure and reflection coefficient (e.g. a face). This problem is present also when using conventional video cameras, as light from e.g. a window may be reflected in the object, and thereby cause difficulties in the detection. However, this problem is further emphasized when exposing the object with active radiation, as use of reflected waves is the basic principle underlying the whole idea. When the reflection is disturbed, or varies over the object, the processing of images is affected negatively.

Also, one of the purposes with introducing reflection based detection is to enable different types of eye detection through sunglasses. Unfortunately, any type of glasses or visors present one of the most significant sources of disturbing reflexes, thus making successful detection through sunglasses difficult still.

Eye detection is one field of technology, in which the problem with disturbing reflexes is present. This technology is widely explored in different situations, including detection of gaze direction for vision controlled computer applications. In order to enable a computer processor to automatically detect different characteristicts of the eyes of a user, image capturing devices are sometimes used to provide a flow of digital images to the processor. To determine the required information, such as the gaze direction, advanced image processing is required, and high quality images, with little or no disturbing features, is of course desirable.

The above problems are especially troublesome in this field of technology, as the requirements on the image quality in some cases are very high. Also, the image capturing is often taking place close to a window or in other situations with varying light conditions, or in darkness. Further, the face of a user can present extremely varying reflective qualities, due to topology and complexion. Finally, users are often wearing glasses, and even more disturbing, sunglasses, hiding the eyes. If the purpose of the image capturing is to detect information about the eyes of a user, such as gaze direction, based on images of his/her face, these problems can be serious.

The article "Improved video-based eye-gaze detection method", by Yoshinobu Ebisawa, IEEE Transactions on instrumentation and measurements, Vol 47, No 4, August 1998, describes a method for acquiring images using two light sources, and subtracting the images from one another in order to obtain improved pupil detection.

Document EP-A-1 125 544 , falling under Art. 54(3) EPC, discloses an apparatus for determining position of an eye having two IR emitter-detector pairs alternately running and configured to minimize on-axis images.

Summary of the invention

In the following description some terms are used with special meanings, defined as follows:

    Object: any object of which an image is desired. The object may be a human being, but the invention may also be applied to other "dead" objects.
    Image: Electronic (digital or analog) representation of an object. Note that an image in this sense not necessarily is intended to be seen by a human, but might only be used to acquire certain information, such as a coordinate point.

Reflex: Unless otherwise stated, the term reflex is used for high intensity reflections from areas with enhanced reflection coefficient on an object, that disturb the image quality. Examples are reflections from glasses, from teeth, polished surfaces, etc. This undesired reflection should not be confused with the "diffuse" reflection of incident light used in all image capturing. Most often in the following description, the disturbing reflexes arise from the active radiation.

Eye detection: Determination of different characteristics of the eyes of a user, such as eye position, direction of gaze, eyelid closure factor or bright eye effect. In some cases the eye detection comprises detection of features neighboring the eyes, such as eyebrows or eye corners.

The object of the present invention is to overcome the described problems in image capturing, and to succeed in acquiring an image of satisfactory quality even in difficult light conditions or darkness, and even when reflexes from e.g. glasses are present in the face of the user.

A second object of the invention is to enable acquiring an image of the eyes of a user that is wearing sunglasses, a visor, or the like.

These objects are achieved with a device according to cl.1.

According to the present invention, the wave emitter provides alternating illumination of the object, and the image capturing device can thus acquire images based on reflections of waves emitted from different angles. These images will present different patterns of enhanced reflections, or reflexes, caused by the waves from the emitter. For example, reflexes from glasses a person is wearing, caused by the emitted waves, will be located in different places in images of the face acquired when the waves are emitted from different points. This fact can be used advantageously in the subsequent treatment, where the disturbing reflexes from the emitted waves can be eliminated or reduced, for example by comparing the images and eliminating areas with increased intensity.

The distance between said points is preferably 2-6 times greater than the cross measure of the aperture (s) of said wave emitter. However, the distance is dependant upon the optics of the image capturing device, and with the development of this technology, other distances might be found advantageous.

According to a first embodiment of the present invention, the wave emitter comprises at least two wave sources located in different points of said at least two points, and arranged to be activated alternately. This eliminates the need for moving parts, but of course requires several wave sources. If the waves sources are inexpensive and easy to activate/deactivate, this embodiment is normally preferred, as the stationary wave sources secure consistent images.

According to a further embodiment of the invention, the wave emitter comprises one wave source and wave guiding means to alternately direct waves from said wave source along different paths. This reduces the number of required wave sources, to the cost of wave guiding means such as mirrors and shutters.

According to a further embodiment of the invention, the wave emitter includes only one wave source, which is arranged to be movable between said at least two points. A possible way to achieve this is to arrange a wave source continuously movable on a closed trajectory, e.g. a circle. The wave source can be arranged to be activated in suitable, different points or, alternatively, the source is activated continuously, and said image capturing device is arranged to capture images when the wave source is located in suitable points. In any case, the intended alternating illumination of the object is accomplished.

The device comprises first processing means, adapted to receive at least two images, acquired with active radiation from different points, and to generate a reflex reduced image. In a first process, it is reflexes from the active radiation that are reduced or eliminated, but by further processing, also other reflexes can be reduced. Thereby a complete system for generating reflex reduced images is realized. This system can be used in a wide variety of applications.

The wave emitter is normally synchronized with the image capturing device, and timing may be non-periodical. However, often a periodic image capturing is implemented, with an alternating frequency dependent upon the application, for example type of video format. In some cases, a frequency of 10-70 Hz may be preferred. In other cases, a higher frequency of more than 100 Hz may be preferred.

The wave emitter is preferably arranged to emit IR waves, and can then be of LED type. An advantage with IR waves is that they do not blind the user nor affect his night vision, and also that they consist of waves with wave lengths outside the visible range, and thus are less affected by other light sources, especially light sources with wave lengths restricted to the visible field, such as fluorescent lamps.

The object of which images are captured can be a human face, and the device according to the invention can then be used in the process of eye detection of a person. Such a device further comprises second processing means, adapted for processing images from said image capturing device and determining required characteristics from said images. This is a field of technology in which actively radiated, reflex reduced images can be of great use, and significantly improve the quality of detection. One type of eye detection of special interest is determination of direction of gaze, but other eye characteristics are also possible.

Brief description of the drawings

A currently preferred embodiment of the present invention will now be described in more detail, with reference to the accompanying drawings.

Fig 1 is a schematic view of an arrangement of a device according to a first embodiment of the invention.

Fig 2a-e is a schematic view of a wave source in fig 1.

Fig 3 is a schematic view of a wave emitter according to another embodiment.

Fig 4 is a schematic view of a wave emitter according to another embodiment.

Fig 5 is a schematic view of a wave emitter according to another embodiment.

Fig 6 is a schematic view of a camera provided with a wave emitter according of the invention.

Fig 7 is a flow chart of a general image acquiring process according to the invention.

Fig 8 is a flow chart of an image acquiring process according to an embodiment of the invention.

Fig 9 is a flow chart of an image acquiring process according to another embodiment of the invention.

Detailed description of a preferred embodiment

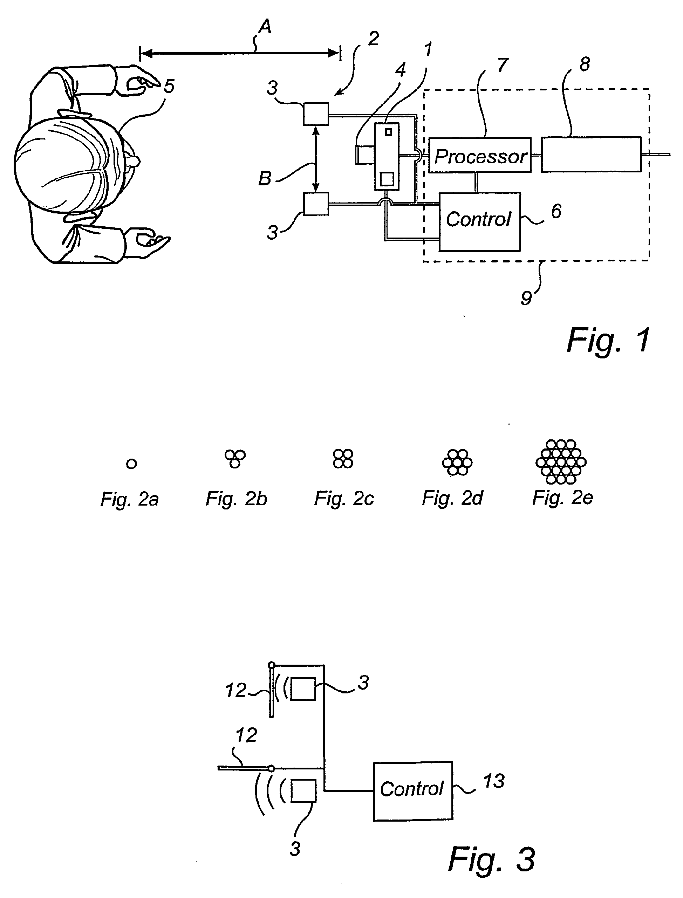

Fig 1 illustrates an arrangement for capturing images of a face 5, with a device according to a first embodiment of the invention, especially adapted for detection of the bright eye effect of the eyes. In this case, a camera 1 with an electronic image sensor is used as image capturing device, and the wave emitter 2 comprises two separate wave sources 3.

The camera is preferably a conventional electronic image sensor camera, either of snapshot type or delivering a stream of consecutive images (i.e. a video camera). The images can be in a digital format, e.g. a bitmap format, or in analog form which then can be converted to a digital format, e.g. using a framegrabber circuit.

The electromagnetic waves emitted by the wave sources can be of different types, including IR radiation. In some cases it is preferred that the waves are within a relatively narrow wave length range outside the range of visible light, and that the camera is provided with a band pass filter 4 corresponding to this range. The influence from the surrounding light is thereby further reduced, as many light sources (computer screens, fluorescent lamps, etc) practically only emit waves in the visible light range. The influence from other sources, e.g. the sun, can be reduced if the total radiated energy in the wave band from the wave emitter is at least a significant fraction of the total sun radiation in the same band.

In conventional arrangements with illumination of an object, quite large wave emitting areas are used, in order to accomplish active radiation with high intensity evenly distributed over the object. In the device according to the invention, however, each wave source has as small aperture as possible, as this requires less separation of the sources for achieving an illumination of the object from one source which is distinguishable from illumination from another source. In conventional arrangements where LEDs are employed for illuminating an object with IR-radiation, normally more than 20 LEDs are arranged in a rectangular pattern. In an arrangement according to the invention, less LEDs 10 can be enough in each wave source 3. Fig 2a-e illustrates some different suitable LED patterns, with the number of LEDs ranging from one (fig 2a) to 19 (fig 2e).

In order to achieve a satisfying result, it is important that reflexes arising from illumination from different points are seperably distinguishable by the image capturing device. Apart from the rather small wave emitting area discussed above, the separation is dependent upon the ability of the camera to distinguish separate high intensity "point" sources of radiation.

Returning to fig 1, the wave sources each consist of three LEDs (according to fig 2b) and are arranged at a distance A of about 50 cm from the face, spaced apart a distance B 4-5 cm. This distance B roughly corresponds to 3 times the cross measure of each wave source area, and has been found to satisfy the requirement of reflex separation, still being close enough to the camera axis to result in the bright eye effect. If the bright eye effect is not required, the two sources may be separated a greater distance.

The wave emitter 2 further comprises a control unit 6 to alternately illuminate the face 5, whereby any disturbing reflexes from the emitted waves in the face appear at slightly different places. The wave sources 3 is synchronized with the camera 1, preferably by the same control unit 6, and acquires one image frame for each illumination. These images can then be treated by processing means 7, such as a computer processor or especially designed hardware, for reducing the reflexes.

The control of the wave sources can be performed by alternately turning the sources on and off, easily accomplished when using LEDs and schematically illustrated in fig 1. Alternatively, as shown schematically in fig 3, the sources 3 are constantly activated, and mechanical or optoelectrical shutters 12 are arranged in front of the sources and controlled by a controller 13, in the simplest case to alternately open and close the shutters 12.

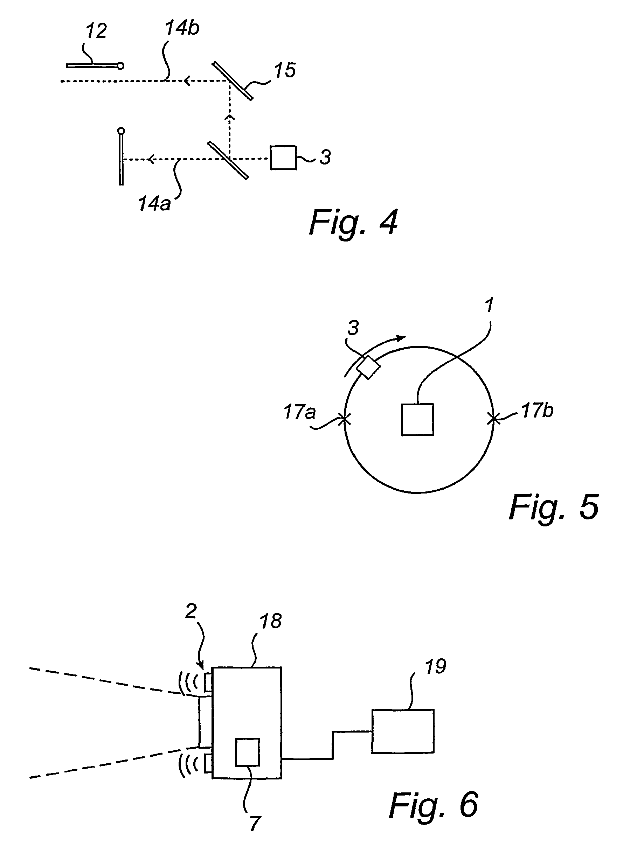

According to a further embodiment of the invention, illustrated in fig 4, only one wave source 3 is used, and two different paths 14a, 14b for the waves are provided, for example by using mirrors 15 and/or beam splitters. Shutters 16 are arranged in front of each path 14, and are controlled in the same way as in the case with two wave sources.

Another possibility, illustrated in fig 5, is to arrange a wave source 3 movable between two points 17a, 17b, so that this single source can illuminate the object from different angles. In the illustrated example, which is showed as viewed from the user, the wave source 3 is arranged to be movable along a trajectory surrounding the camera 1. The camera can then be controlled to acquire images every time the wave source is located in e.g. one of the points 17a, 17b.

As mentioned above, the images captured by the camera are in the illustrated example fed to a processing device 7, adapted for treating the images in order to generate a reflex reduced image.

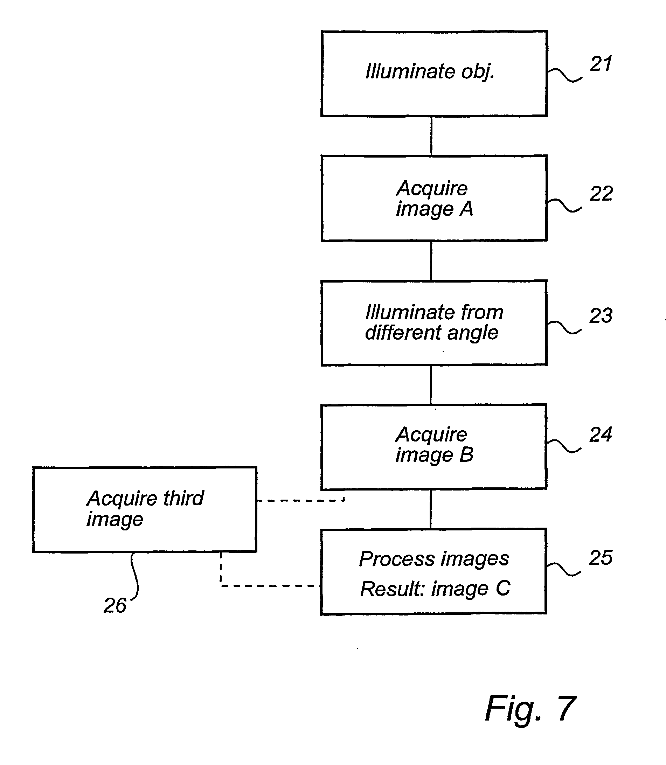

The image acquiring process is illustrated in fig 7. First, in step 21, the object is illuminated with electromagnetic waves emitted from a first angle, and a first image is acquired, step 22. Then, in step 23, the illumination is altered in any of the ways described above, resulting in waves emitted towards the object from a different angle, and a second image is acquired in step 24. The two images are processed in step 25, resulting in an image C in which reflexes from the active radiation have been reduced.

The processing of the images can be of different kinds. Basically, two images, acquired with illumination from different angles, are compared to each other to result in an improved image where disturbing reflexes from the active radiation have been reduced. The reduction can be accomplished by building the improved image of information from either the first or second images, systematically selecting the information that has least intensity. The processing device 7 preferably comprises a computer processor, programmed to perform a pixel by pixel comparison between different images. Note that the word pixel is used as "picture element" not necessarily implying a digital image format. Both analog and digital signals may be used, and the comparison can be performed serially or in parallel, in hardware or in software.

A simple formula implementing this method could read as follows:

$$\mathrm{I\,(C_n)\,=\,MIN\,(I\,(A_{n+c})\,,\ \ I\,(B_{n+d})\,)\,,}$$

where I(x) is the intensity in pixel x, $A_n$ is pixel n in the first acquired image A, $B_n$ is pixel n in the second acquired image B, $C_n$ is pixel n in the improved image C, and c and d are offset factors for adjustments between the different images, e.g. if cameras with different characteristics are used for different images.

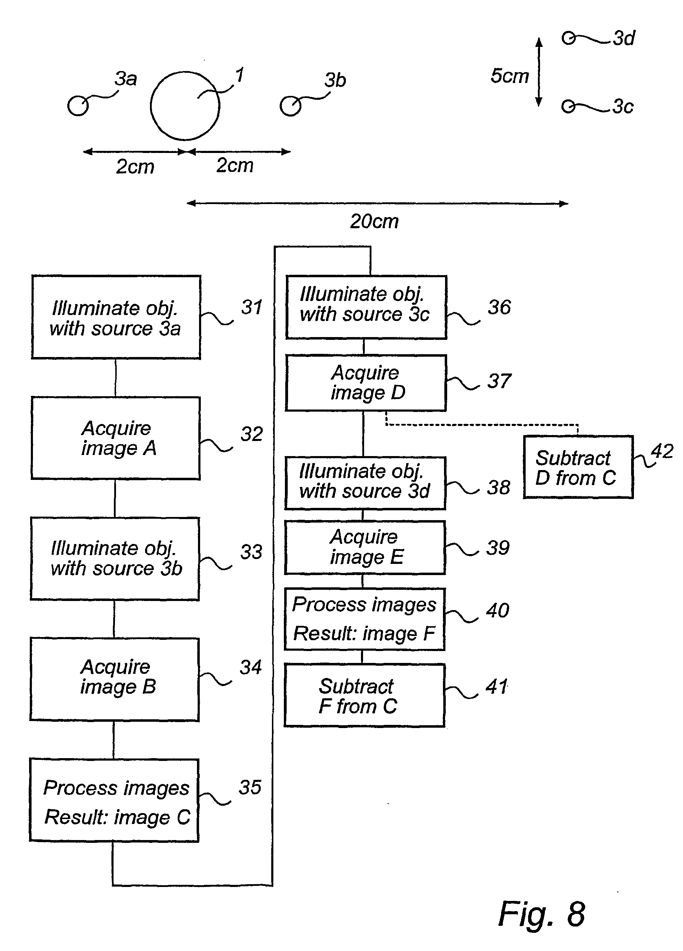

To achieve an improved result, and especially to reduce also other disturbing reflexes, it can sometimes be advantageous to acquire additional images in an additional step 26. Two different implementations of this method will be described in more detail with reference to the figures 7 and 8.

In the first case, illustrated in fig 8, the purpose of the image acquiring is detection of bright eye effect, and two wave sources 3a, 3b are arranged as shown in fig 1, separated approx. 4 cm, with the camera in the middle. Additionally, two wave sources 3c, 3d are provided at a greater distance from the camera, for example 20 cm away. In the illustrated example, these sources 3c and 3d are located on the same side of the camera, and are separated 5 cm. In fig 7, the first sources 3a, 3b are arranged in the horizontal plane and the additional sources 3c, 3d in the vertical plane, but this is not necessary.

Steps 31-35 are equivalent to steps 21-25 in fig 7, and results in an image C with reduced reflexes from the active radiation, as described above. As the sources 3a and 3b are so close to the camera 1, a bright eye effect will be present in the images A, B and C. In step 36, the object is radiated from wave source 3c, and an image D is acquired in step 37. Then, in step 38, active radiation is provided from source 3d, and an image E is acquired in step 39. These later images D and E are then processed in step 40, to result in a second reflex reduced image F, this time without bright eye effect. In a final step 41, the image F is subtracted from image C.

The most significant difference between images C and F is the bright eye effect present in image C. The subtraction of these images will thus remove most of the image, including disturbing reflexes from the diffuse radiation, leaving only the bright eye effect. This enables a quick and precise determination of the position of the eyes.

In a more simple alternative method, the steps 38-41 are substituted with step 42, in which image D is subtracted from image C. In this case, the reflexes from the active radiation is not removed from the image without bright eye effect (image D). Therefore, the final image will comprise some patterns apart from the bright eye effect. In some cases, this simpler method is sufficient to locate the eyes.

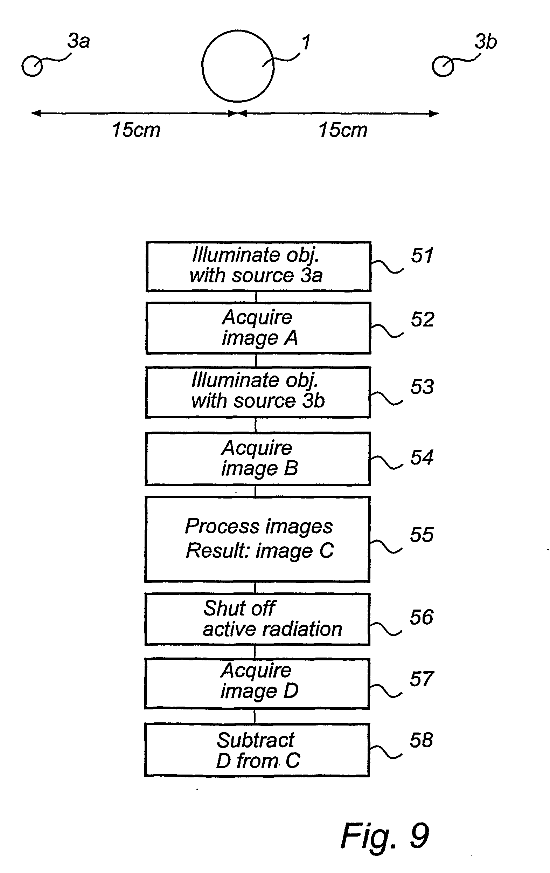

In the second case, illustrated in fig 9, the purpose of the image acquiring is not to detect the eyes, but only to acquire an image of the object without disturbing reflexes from the active radiation nor fromn the ambient light. The wave sources 3a, 3b are here arranged at a slightly larger distance from the camera 1, as no bright eye effect is required.

Steps 51-55 are equivalent to steps 31-35 above, resulting in an image C with reduced reflexes from the active radiation. Then in step 56, the active radiation is deactivated, and an image D is acquired in step 57. This image D is then subtracted from the image C to generate a final image.

As images C and D comprises the same image information resulting from the ambient light(sunlight, indoor illumination, etc) the contribution from these sources, including disturbing reflexes, will be eliminated in the resulting image.

It is not necessary that reflex reduction be performed for every image in a sequence. Depending on the field of application, it might be sufficient to apply the reflex reduction only to one image frame out of an image sequence, or on chosen still pictures.

In fig 1, the device is illustrated with the wave emitter separated from the image capturing device. Of course, it is possible to provide a conventional electronic image sensor camera 18 (video or snap shot), with a wave emitter 2 according to the invention, as illustrated in fig 6. The camera 18 can further be provided with a memory 19 (internal in the image capturing device, or external), for storing images. In some cases it might be advantageous to store the original pictures, acquired with different illumination according to the invention, for later processing. In other cases, it might be preferred to perform the reflex reduction and to store the reflex reduced images. In this case, the camera is also provided with processing means 7 (hardware or software), thereby providing a camera with reflex reduction capacity.

One field of application, particularly suitable for the device according to the invention, is the field of eye detection.

In this case, the reflex reduced images are further processed in a second processing means 8, preferably a suitably programmed computer processor. The processor analyzes the images and determines the required characteristics, for example the location of the iris of the user, or to the direction of gaze by relating the location of the iris to the other points of the face. In processes such as these, the reflex reduced images acquired with the present invention improve the quality of the results.

Note that in figure one, the processing devices 7 and 8, and the control unit 6 are illustrated schematically as separate units. Normally, however, these units are integrated in one processing device, such as a personal computer, illustrated by the dashed box 9 in fig 1.

In the figures, and in the description above, the number of points from which waves are emitted have been limited to two. This is not to be regarded as a limitation of the invention, and more points can very well be advantageous, further extending the reflex reduction capacity as mentioned above.

When using more than one camera, the minimum is still two different points for wave emitting. It may be advantageous to implement a two-camera system, with at least one wave source provided in each camera.

Also, it may be advantageous to allow for combinations of illumination from several wave sources. For example, the intensity of the electromagnetic waves are normally equal in the two sources. However, two sources can be controlled to illuminate with different intensities. Further, if the active radiation is restricted to a certain wave band, this band can be varied depending on the situation.

**Claims**

1. A device for acquiring a reflex reduced image of an object, said object being a human face (5), comprising
   at least one wave emitter (2) for emitting electromagnetic waves towards the object alternatingly from at least two points,
at least one image capturing device (1), directed towards the object and arranged to capture a first image when waves are emited from a first point, and a second image when waves are emitted from a second point,
first processing means (7), adapted to receive said first and second images and to generate the reflex reduced image
**characterized in that**
said points are separated such a distance (B) that disturbing reflexes, resulting from waves emitted from the wave emitter and appearing in said first and second images, are separably distinguishable by the image capturing device (1), and
said reflex reduced image is processed using second processing means (8) for eye detection of a person, thereby allowing for determining the direction of gaze of the person.

2. Device according to claim 1, wherein each pixel of the reflex reduced image contains image information deduced from either said first or said second image.

3. Device according to claim 2, wherein said first processing means (7) is adapted to perform a pixel-by-pixel comparison of said first and second images, and, for each pixel, to select the image information, having the least intensity to be used for generating the reflex reduced image.

4. Device according to claim 1 - 3, wherein said distance (B) is 2-6 times greater than the cross measure of the aperture(s) of said wave emitter.

5. Device according to any of the preceding claims, wherein said wave emitter (2) comprises at least two wave sources (3) located in different points of said at least two points, and a control unit (6) arranged to activate said wave sources (3) alternately.

6. Device according to any of the preceding claims, wherein said wave emitter comprises one wave source (3) and wave guiding means (15) to alternately direct waves from said wave source along different paths (14a, 14b).

7. Device according to any of the preceding claims, wherein said wave emitter (2) comprises one wave source (3) arranged to be movable between said at least two points (17a, 17b).

8. Device according to any of the preceding claims, wherein said wave emitter (2) is synchronized with said image capturing device (1).

9. Device according to any of the preceding claims, wherein said wave emitter (2) is arranged to alternate with a frequency of 10-70 Hz.

10. Device according to any of the preceding claims, wherein said wave emitter (2) is arranged to emit IR-waves.

11. Device according to any of the preceding claims, wherein said wave emitter (2) include at least one LED (10).

## Patentansprüche

1. Ein Gerät für den Erwerb einer Reflex verringert Bild eines Objekts, sagte Objekt wird ein menschliches Antlitz (5), bestehend aus
mindestens eine Welle Emitter (2) zu emittierenden elektromagnetischen Wellen auf das Objekt abwechselnd aus mindestens zwei Punkte,
mindestens eine Bilderfassung Gerät (1), die auf das Objekt und angeordnet, um das erste Bild, wenn emittierten Wellen sind aus einer ersten und einer zweiten Bild, wenn Wellen emittiert werden von einem zweiten Punkt,
Mittel der ersten Bearbeitung (7), angepasst zu erhalten, sagte ersten und zweiten Bilder und ein Reflex verringert Bild,
**dadurch gekennzeichnet, dass**
Punkte getrennt sind, sagte eine solche Distanz (B), die störende Reflexe, die sich aus Wellen von Sender und die Welle, die in dieser ersten und zweiten Bilder sind voneinander trennbar von der Bilderfassung Gerät (1),
sagte Reflex verringert Bild wird mit Hilfe zweite Verarbeitung bedeutet (8) für Auge Erkennung einer Person, so dass für die Bestimmung der Richtung der Blick der Person.

2. Gerät nach Anspruch 1, bei der ersten Bearbeitung Mittel (7), angepasst zu erhalten, sagte ersten und zweiten und Bilder zu erzeugen, der Reflex verringert Bild jedes Pixel des reflektierten reduziert Bild Bild enthält Informationen aus entweder sagte ersten oder zweiten Bild gesagt.

3. Gerät nach Anspruch 2, **dadurch** sagte Erstverarbeitung bedeutet (7) angepasst ist, um eine Pixelby-Pixel-Vergleich der genannten ersten und zweiten Bilder, und für jedes Pixel, das Bild, um Informationen, die am wenigsten Intensität verwendet werden für die Generierung der Reflex verringert Bild.

4. Gerät nach Anspruch 1 - 3, **dadurch** sagte Distanz (B) ist 2 - 6 mal größer als das Kreuz Maßnahme der Blende (n) der genannten Welle Sender.

5. Gerät nach einem der vorhergehenden Ansprüche, **dadurch** sagte Welle Emitter (2) besteht aus mindestens zwei Quellen Welle (3) in verschiedenen Punkten der genannten mindestens zwei Punkte, und eine Steuereinheit (6) angeordnet zu aktivieren, sagte Welle Quellen (3) abwechselnd.

6. Gerät nach einem der vorhergehenden Ansprüche, **dadurch** sagte Welle besteht aus einem Sender Welle Source (3) und Welle Führung bedeutet (15) abwechselnd auf direkte Wellen Welle sagte Quelltext zusammen verschiedene Pfade (14a, 14b).

7. Gerät nach einem der vorhergehenden Ansprüche, **dadurch** sagte Welle Emitter (2) besteht aus einer Welle Source (3) angeordnet werden, sagte beweglichen zwischen mindestens zwei Punkten (17a, 17b).

8. Gerät nach einem der vorhergehenden Ansprüche, **dadurch** sagte Welle Emitter (2) ist synchronisiert mit sagte Bilderfassung Gerät (1).

9. Gerät nach einem der vorhergehenden Ansprüche, **dadurch** sagte Welle Emitter (2) ist zu wechseln sich ab mit einer Frequenz von 10-70 Hz.

10. Gerät nach einem der vorhergehenden Ansprüche, **dadurch** sagte Welle Emitter (2) ist die Emission von IR-Wellen.

11. Gerät nach einem der vorhergehenden Ansprüche, **dadurch** sagte Welle Emitter (2) mindestens eine LED (10).

## Revendications

1. Un dispositif pour l'acquisition d'une reflex image réduite d'un objet, dit objet étant un visage humain (5), comprenant
au moins un émetteur d'ondes (2) pour l'émission des ondes électromagnétiques vers alternant l'objet d'au moins deux points,
au moins un dispositif de capture d'image (1), dirigé vers l'objet et aménagé pour la capture d'une première image lorsque les vagues sont émis à partir d'un premier point, et une seconde image lorsque les ondes sont émises à partir d'un second point,
premier moyen de traitement (7), adapté à recevoir ladite première et deuxième images et de générer un réflexe d'image réduite,
**caractérisé en ce que**
dit points sont séparés d'une telle distance (B) que les réflexes inquiétant, résultant d'ondes émis par l'émetteur d'ondes et qui figurent dans ladite première et deuxième images, séparable sont distingués par le dispositif de capture d'image (1), et
dit reflex image réduite sont traitées en utilisant deuxième moyen de traitement (8) pour la détection des yeux d'une personne, permettant ainsi de déterminer la direction du regard de la personne.

**2.** Dispositif selon la revendication 1, dans lequel la première transformation des moyens (7), adapté à recevoir ladite première et deuxième images et de générer le réflexe d'image réduite chaque pixel de l'image réduite reflète l'image contient des informations, soit déduite de ladite première ou ladite seconde image.

**3.** Dispositif selon la revendication 2, **caractérisé en ce que** la première transformation des moyens (7) est adapté pour effectuer un pixel par pixel a dit la comparaison des première et deuxième images, et, pour chaque pixel, l'image pour sélectionner des informations en ayant le moins d'intensité pour être utilisé pour générer le réflexe d'image réduite.

**4.** Dispositif selon la revendication 1 - 3, **caractérisé en ce que** la distance (B) est 2 - 6 fois supérieure à la mesure de traverser l'ouverture (s) de cette vague de l'émetteur.

**5.** Dispositif selon l'une quelconque des précédentes revendications, **caractérisé en ce que** l'émetteur d'ondes (2) comporte au moins deux sources d'onde (3) situés dans différents points de ladite au moins deux points, et une unité de contrôle (6) a organisé à activer des sources d'ondes (3) en alternance.

**6.** Dispositif selon l'une quelconque des précédentes revendications, **caractérisé en ce que** l'émetteur d'ondes comporte une vague source (3) et les moyens de guidage d'onde (15) à tour de rôle direct à partir de vagues le long de ladite source d'onde différentes voies (14a, 14b).

**7.** Dispositif selon l'une quelconque des précédentes revendications, **caractérisé en ce que** l'émetteur d'ondes (2) comporte une source d'ondes (3) disposés à être mobiles entre dit au moins deux points (17a, 17b).

**8.** Dispositif selon l'une quelconque des précédentes revendications, **caractérisé en ce que** l'émetteur d'ondes (2) est synchronisé avec ledit appareil de capture d'image (1).

**9.** Dispositif selon l'une quelconque des précédentes revendications, **caractérisé en ce que** l'émetteur d'ondes (2) est organisée en alternance avec une fréquence de 10-70 Hz.

**10.** Dispositif selon l'une quelconque des précédentes revendications, **caractérisé en ce que** l'émetteur d'ondes (2) est disposé à émettre des ondes IR.

**11.** Dispositif selon l'une quelconque des précédentes revendications, **caractérisé en ce que** l'émetteur d'ondes (2) comporte au moins une LED (10).

Fig. 1

Fig. 2a   Fig. 2b   Fig. 2c   Fig. 2d   Fig. 2e

Fig. 3

*Fig. 4*

*Fig. 5*

*Fig. 6*

Fig. 7

3a $\qquad$ 1 $\qquad$ 3b $\qquad$ 3d

5cm $\uparrow$ $\downarrow$

3c

2cm $\quad$ 2cm

20cm

| Illuminate obj. with source 3a | 31 |
| Acquire image A | 32 |
| Illuminate obj. with source 3b | 33 |
| Acquire image B | 34 |
| Process images Result: image C | 35 |

| Illuminate obj. with source 3c | 36 |
| Acquire image D | 37 |
| Illuminate obj. with source 3d | 38 |
| Acquire image E | 39 |
| Process images Result: image F | 40 |
| Subtract F from C | 41 |

Subtract D from C — 42

## Fig. 8

*Fig. 9*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1125544 A

**Non-patent literature cited in the description**

- **Yoshinobu Ebisawa.** Improved video-based eye-gaze detection method. *IEEE Transactions on instrumentation and measurements,* August 1998, vol. 47 (4